# EUROPEAN PATENT APPLICATION

(11) **EP 1 972 633 A1**
(43) Date of publication of application: **24.09.2008**
(21) Application number: 06841739.3
(22) Date of filing: 14.12.2006
(51) Int. Cl.: C07D 493/18, C07D 493/22, A61K 31/357, A61P 35/00, C07D 311/00, C07D 323/00

(54) **ANTITUMOUR COMPOUNDS**

(30) Priority: 15.12.2005 ES 200503092
(71) Applicant: PHARMA MAR, S.A., 28770 Colmenar Viejo (Madrid) (ES)
(72) Inventor: MARTÍN LÓPEZ, Mª Jesús, E-28770 Colmenar Viejo - Madrid (ES); REYES BENÍTEZ, José Fernando, E-28770 Colmenar Viejo - Madrid (ES); FERNÁNDEZ RODRÍGUEZ, Rogelio, E-28770 Colmenar Viejo - Madrid (ES); FRANCESCH SOLLOSO, Andrés, E-28770 Colmenar Viejo - Madrid (ES); CUEVAS MARCHANTE, María del Carmen, E-28770 Colmenar Viejo - Madrid (ES); COELLO MOLINERO, Laura, E-28770 Colmenar Viejo - Madrid (ES)
(74) Representative: ABG Patentes, S.L.
(86) International application number: PCT/ES2006/000687
(87) International publication number: WO 2007/068776

(57) **Abstract**

The invention relates to novel antitumor compounds of general formula 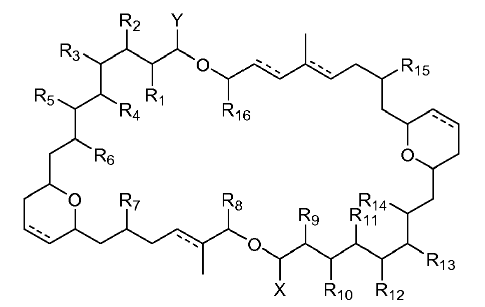 as well as their corresponding pharmaceutically acceptable salts, derivatives, prodrugs and stereoisomers. These compounds can be obtained by isolating a sponge from family *Theonellidae,* genus *Theonella* and species *swinhoei,* and forming derivatives from the isolated compounds. These compounds have cytotoxic activity and can be used for the treatment of the cancer.

## Description

### Field of the Invention

The present invention relates to novel antitumor compounds, to pharmaceutical compositions containing them and to their use as antitumor agents.

### Background of the Invention

Several symmetrical dimeric macrolides have been shown to have antitumor, antiviral and/or antifungal properties. Specifically, Kitagawa et al. (Chem. Pharm. Bull., 1994, 42(1), 19-26) isolated several symmetrical dimeric macrolides of the Okinawan marine sponge *Theonella swinhoei:*

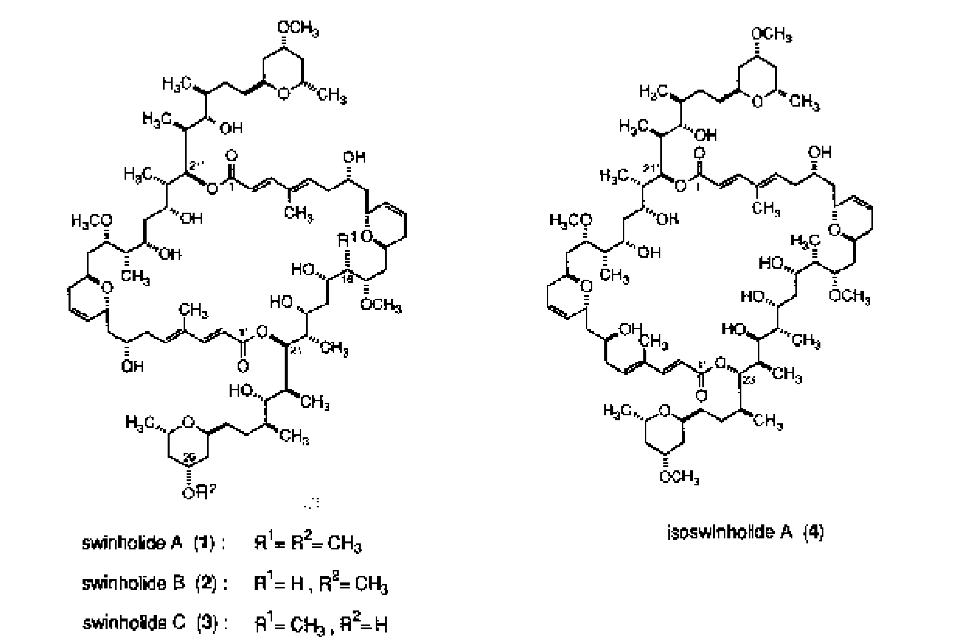

Swinholide A (1), B (2) and C (3) have shown to have a potent cytotoxicity against L1210 and KB cells with IC₅₀ values of 0.03, 0.30 and 0.14 µg/mL (for L1210) and 0.04, 0.04 and 0.05 µg/mL (for KB), respectively. Nevertheless, it was observed that isoswinholide A (4) showed lower cytotoxicity [IC₅₀ 1.35 µg/mL (L1210) and 1.1 µg/mL (KB)] than the other previously mentioned analogs.

Kitagawa *et al.* also examined the cytotoxicity of several dimers derived from Swinholide A (1): 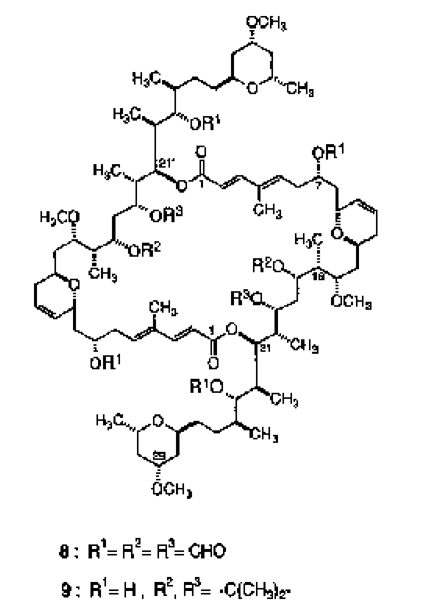 observing that both dimers (8 and 9) show scarce growth inhibitory power in KB cells (51.1% inhibition at 50 µg/mL and 19.3% inhibition at 10 µg/mL, respectively).

Other dimeric macrolides which were obtained from Swinholide A (1) were the following:

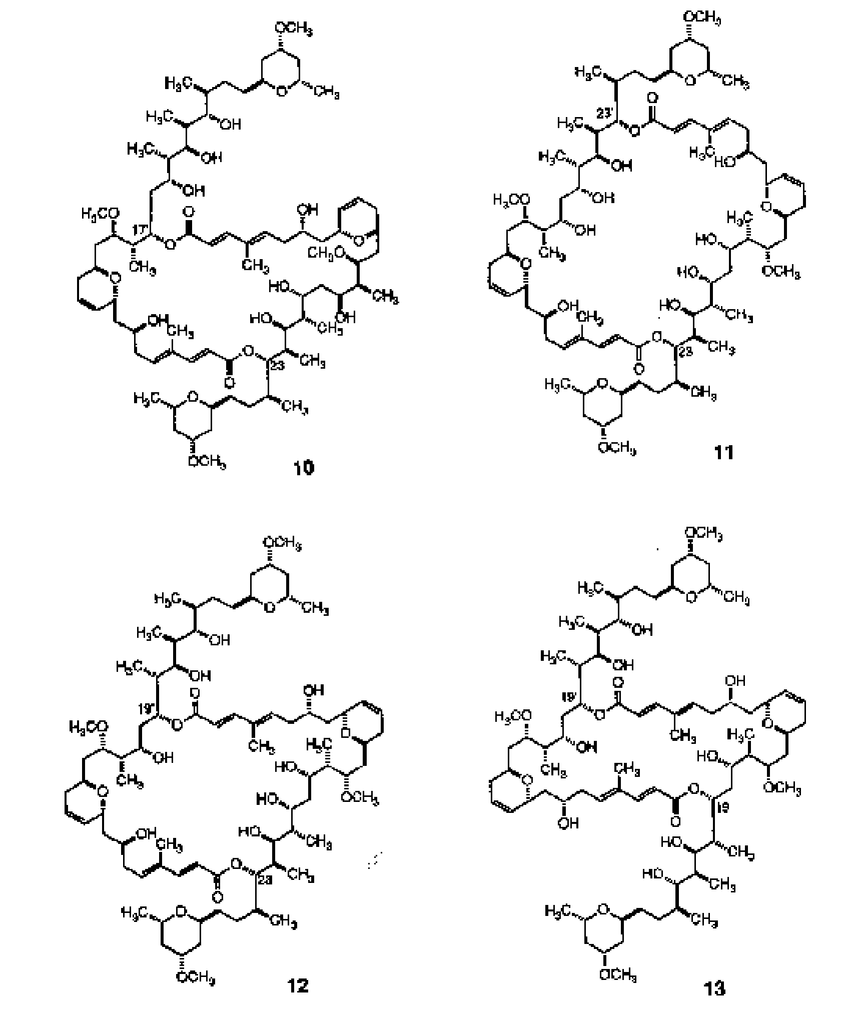

The cytotoxicity of these compounds (10-13) against L1210 and KB cells is less than the cytotoxicity shown by Swinholide A (1).

In parallel, Kitagawa et al. examined the antitumor effects of Swinholide A (1) and its isomers against P388 leukemia in CDF1 mice. It was unexpectedly observed that Swinholide A (1), isoswinholide A (4) and the isomer (11) were toxic and did not show promising antitumor activity.

In addition, patent application WO 88/00195 describes the following compounds (Misakinolide A (14) and derivatives (15)), which were extracted from a marine sponge of the genus *Theonella:*

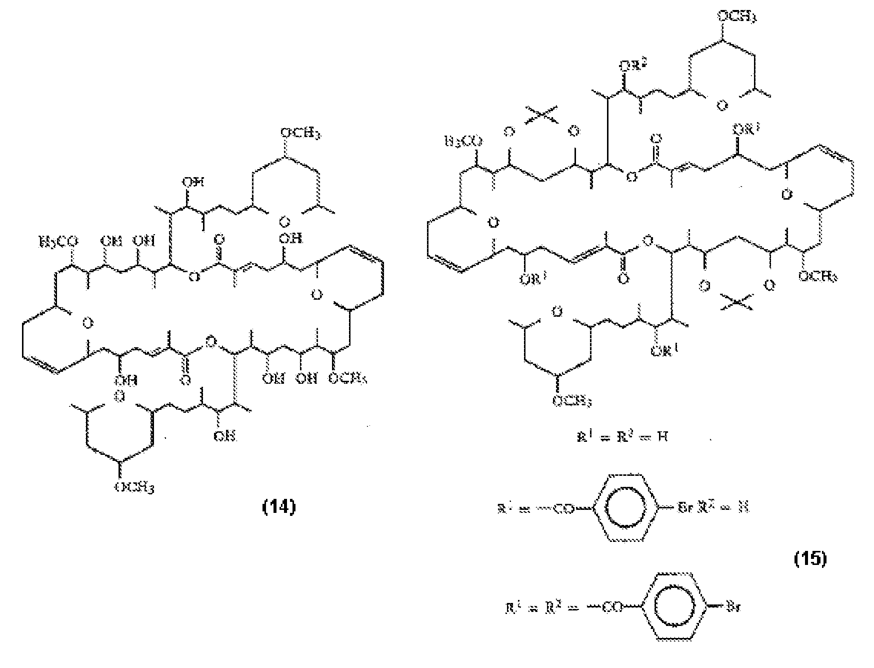

In said patent application, *in vitro* antitumor activity of Misakinolide A (14) against P388, HCT-8, A549 and MDA-MB-231 cancer cells is described. Likewise, it has also been described that in addition to having a potent cytotoxicity [IC₅₀ 0.035 µg/mL (L1210)], Misakinolide A also has antitumor activity [T/C 140% at a dose of 0.1 mg/kg (mouse) against P388 leukemia] (Chem. Pharm. Bull., 1994, 42(1), 19-26).

Cancer continues to be one of the main causes of death among the animal species and humans. Great efforts for finding safe and effective novel antitumor agents which contribute to increasing the therapeutic arsenal needed for the effective treatment of patients with this disease have been made and continue to be made. In this sense, the present invention aims to solve this problem, providing novel compounds useful in the treatment of cancer.

### Summary of the Invention

The present invention is aimed at asymmetric dimeric macrolides with general formula I, as well as their corresponding pharmaceutically acceptable salts, derivatives, prodrugs and stereoisomers, 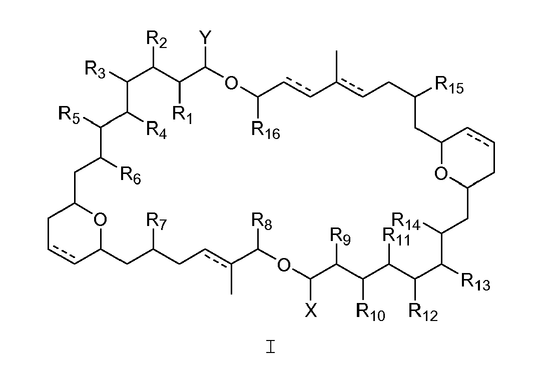 wherein,
R₁-₁₆ are groups independently selected from hydrogen, protected or non-protected hydroxyl, substituted or non-substituted C₁-C₂₄ alkyl, substituted or non-substituted C₂-C₂₄ alkenyl, substituted or non-substituted C₂-C₂₄ alkynyl, =0, ORₐ, OCORₐ, NRₐR_{b}, NRₐCOR_{b}, CONRₐR_{b}, CORₐ, COORₐ and halogen.
X and Y are groups independently selected from substituted or non-substituted C₁-C₂₄ alkyl, substituted or non-substituted C₂-C₂₄ alkenyl, substituted or non-substituted C₂-C₂₄ alkynyl, =O, ORₐ, OCORₐ, NRₐR_{b}, NRₐCOR_{b}, CONRₐR_{b}, CORₐ, COORₐ and halogen;
Rₐ and R_{b} are groups independently selected from hydrogen, halogen, substituted or non-substituted C₁-C₁₂ alkyl, substituted or non-substituted C₂-C₁₂ alkenyl, substituted or non-substituted C₂-C₁₂ alkynyl, substituted or non-substituted aryl and substituted or non-substituted heterocycle; and
the dotted line represents the optional presence of a double bond.

The present invention also relates to isolating compounds of formula I from a sponge of the family Theonellidae, genus *Theonella* and species *swinhoei*, and to forming derivatives from the isolated compounds.

In addition, the present invention also relates to a compound of general formula I or a pharmaceutically acceptable salt, a derivative, a prodrug or a stereoisomer thereof, for its use as a medicament.

The invention is likewise aimed at the use of a compound of formula I or a pharmaceutically acceptable salt, a derivative, a prodrug or a stereoisomer thereof for the preparation of a medicament aimed at the treatment of cancer.

The present invention equally relates to pharmaceutical compositions comprising a compound of formula I or one of its corresponding pharmaceutically acceptable salts, derivatives, prodrugs or stereoisomers in mixture with a pharmaceutically acceptable excipient or diluent.

### Detailed Description of the Invention

The compounds object of the present invention correspond to asymmetric dimeric macrolides of general formula I 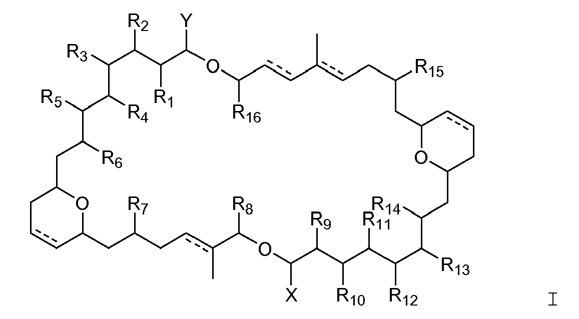 wherein,
R₁-₁₆ are groups independently selected from hydrogen, protected or non-protected hydroxyl, substituted or non-substituted C₁-C₂₄ alkyl, substituted or non-substituted C₂-C₂₄ alkenyl, substituted or non-substituted C₂-C₂₄ alkynyl, =0, ORₐ, OCORₐ, NRₐR_{b}, NRₐCOR_{b}, CONRₐR_{b}, CORₐ, COORₐ and halogen.
X and Y are groups independently selected from substituted or non-substituted C₁-C₂₄ alkyl, substituted or non-substituted C₂-C₂₄ alkenyl, substituted or non-substituted C₂-C₂₄ alkynyl, =O, ORₐ, OCORₐ, NRₐR_{b}, NRₐCOR_{b}, CONRₐR_{b}, CORₐ, COORₐ and halogen;
Rₐ and R_{b} are groups independently selected from hydrogen, halogen, substituted or non-substituted C₁-C₁₂ alkyl, substituted or non-substituted C₂-C₁₂ alkenyl, substituted or non-substituted C₂-C₁₂ alkynyl, substituted or non-substituted aryl and substituted or non-substituted heterocycle; and
the dotted line represents the optional presence of a double bond.

In these compounds the groups or substituents can be selected in accordance with the following criteria:

The term alkyl represents a linear or branched carbon chain having 1 to 24 carbon atoms. Alkyl groups of 1 to 6 carbon atoms are preferred, and especially preferred are those made up of 1, 2, 3 and 4 carbon atoms. The methyl, ethyl, propyl, isopropyl, butyl, sec-butyl and tert-butyl groups are especially preferred alkyl groups in the compounds of the present invention. Likewise, as used in the present invention, the term alkyl relates to both a cyclic and a non-cyclic group, taking into account that the cyclic groups will comprise at least three carbon atoms in the ring. Other preferred alkyl groups are those having from 5 to 12 carbon atoms, being especially preferred those made up of 6, 7, 8, 9 and 10 carbon atoms. The hexyl, heptyl, 1,3-dimethylpentyl, octyl, 1,3-dimethylhexyl and nonyl groups are especially preferred alkyl groups in the compounds of the present invention.

The terms alkenyl and alkynyl represent linear or branched unsaturated alkyl chains containing from 2 to 24 carbon atoms and including one or more unsaturations. The alkenyl and alkynyl groups having from 2 to 6 carbon atoms are preferred, and especially preferred are those made up of 2, 3 and 4 carbon atoms. Likewise, the terms alkenyl and alkynyl, as used in the present invention, relate to both cyclic and non-cyclic groups, taking into account that the cyclic groups will comprise at least three carbon atoms in the ring. Other preferred alkenyl and alkynyl groups are those having 5 to 12 carbon atoms.

Among the aryl groups which can be present in the compounds of the invention are those containing one or several rings, including multiple rings with separated or fused aryl or heteroaryl groups. Typically, the aryl groups contain 1 to 3 rings and 4 to 18 carbon atoms in the ring(s). Among the preferred aryl groups are phenyl, naphthyl, biphenyl, phenanthryl and anthracyl, all of them substituted or non-substituted.

Among the heterocycle groups which can be present in the compounds of the invention are both heteroaromatic and heteroalicyclic groups. The heteroaromatic groups contain one, two or three heteroatoms selected from N, O and S and include, for example, groups such as coumarinyl, preferably 8-coumarinyl, quinolinyl, preferably 8-quinolinyl, pyridyl, pyrazinyl, pyrimidyl, furyl, pyrrolyl, thienyl, thiazolyl, oxazolyl, imidazolyl, indolyl, benzofuranyl and benzothiazolyl. The heteroalicyclic groups contain one, two or three heteroatoms selected from N, O, and S and include, for example, groups such as tetrahydrofuranyl, tetrahydropyranyl, piperidinyl, morpholine and pyrrolidinyl. The heterocycle groups can be both substituted and non-substituted.

The previously mentioned groups can be optionally substituted in one or several of their available positions, independently, by one or several suitable substituents, such as OR', =O, SR', SOR', SO₂R', NO₂, NHR', N(R')₂, =N-R', NHCOR', N(COR')₂, NHSO₂R', NR'C(=NR')NHR', CN, halogen, C(=O)R', COOR', OC(=O)R', CONHR', CON(R')₂, substituted or non-substituted C₁-C₁₂ alkyl, substituted or non-substituted C₂-C₁₂ alkenyl, substituted or non-substituted C₂-C₁₂ alkynyl, substituted or non-substituted aryl and substituted or non-substituted heterocycle, where each group R' is independently selected from H, OH, NO₂, NH₂, SH, CN, halogen, =O, C(=O)H, C(=O)alkyl, COOH, substituted or non-substituted C₁-C₁₂ alkyl, substituted or non-substituted C₂-C₁₂ alkenyl, substituted or non-substituted C₂-C₁₂ alkynyl, substituted or non-substituted aryl and substituted or non-substituted heterocycle. Among the halogen substituents which can be present in the compounds of the present invention F, Cl, Br and I are included. In the case of those groups which in turn are substituted, the corresponding substituent can be chosen from the list of substituents mentioned herein.

The hydroxyl groups can be optionally protected. There is a great number of hydroxyl protecting groups, and they are well known by a person skilled in the art. As a guide, see Protecting groups, Kocienski, 2004, 3^{rd} edition.

The term "pharmaceutically acceptable salts, derivatives, prodrugs" relates to any pharmaceutically acceptable salt, ester, solvate, hydrate or any other compound which, after its administration to the patient, is capable of (directly or indirectly) providing a compound of general formula I. Nevertheless, it must be taken into account that non-pharmaceutically acceptable salts also are within the scope of the invention since they can be useful in the preparation of pharmaceutically acceptable salts. The preparation of the salts, prodrugs and derivatives can be performed by means of methods known in the state of the art.

For example, the pharmaceutically acceptable salts of the compounds of the present invention are obtained from the corresponding compounds having acid or base units, by means of conventional chemical methods. Generally, said salts are prepared, for example, by reacting the corresponding base form or free acid of said compound with a stoichiometric amount of the appropriate base or acid in water, in an organic solvent or in a mixture of both. Usually, the preferred non-aqueous media are ether, ethyl acetate, ethanol, isopropanol or acetonitrile. Included among the acid addition salts are mineral acid addition salts such as hydrochloride, hydrobromide, hydroiodide, sulfate, nitrate and phosphate, and organic acid addition salts such as acetate, trifluoroacetate, maleate, fumarate, citrate, oxalate, succinate, tartrate, malate, mandelate, methanesulfonate and p-toluenesulfonate. Included among the base addition salts are inorganic salts such as sodium, potassium, calcium and ammonium salts, and organic salts such as ethylenediamine, ethanolamine, N,N-dialkylenethanolamine, triethanolamine and basic amino acid salts.

The compounds of the present invention can be in crystalline form, both as free compounds and solvates (for example, hydrates) in which both forms are included within the scope of the present invention. Solvation methods are generally known in the state of the art.

Any compound which is a prodrug of a compound of general formula I is included within the scope of the present invention. The term "prodrug" is used in its broadest meaning and encompasses all those derivatives susceptible of being transformed *in vivo* into any of the compounds of the invention. Any person skilled in the art knows which derivatives can be involved and includes, for example, compounds in which the free hydroxyl group is converted into an ester derivative, or an ester is modified by transesterification or a suitable amide is formed.

The compounds of the present invention represented by the general formula I have more than one stereogenic center, so the invention equally relates to each and every one of the possible enantiomers and diastereoisomers which can be formulated, as well as to the possible Z and E stereoisomers which can be formed when a double bond exists in the molecule. Both pure isomers and mixtures of isomers of said compounds are within the scope of the present invention.

Among the preferred compounds of the present invention are those in which R₁₋₁₆ are groups independently selected from hydrogen, protected or non-protected hydroxyl, ORₐ, OCORₐ, =O, NRₐR_{b}, NRₐCOR_{b}, halogen and substituted or non-substituted C₁-C₂₄ alkyl, where Rₐ has been previously defined. Especially preferred are those compounds in which R₁₋₇ and R₉₋₁₅ are groups independently selected from hydrogen, hydroxyl, C₁-C₆ alkyl and ORₐ; and among those hydrogen, hydroxyl, methyl and methoxyl are preferred. Likewise, it is especially preferred that the R₈ and R₁₆ groups are =O.

Preferably X and Y are groups independently selected from substituted or non-substituted C₅-C₁₂ alkyl and substituted or non-substituted C₅-C₁₂ alkenyl. It is especially preferred that they are independently substituted by one or several suitable substituents, and substituted in particular by one or several of the following substituents: OH, OR', NHCOR' and substituted or non-substituted heterocycle, where R' has been previously defined. More preferably, X and Y are made up of C₅-C₁₂ alkyl groups substituted by one or several hydroxyl groups and/or substituted heterocycles. It is especially preferred when X and Y are

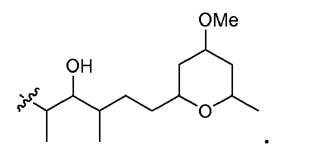

The presence of double bonds in those sites indicated with dotted lines in general formula I is preferable.

The following compound is a compound especially preferred in the present invention:

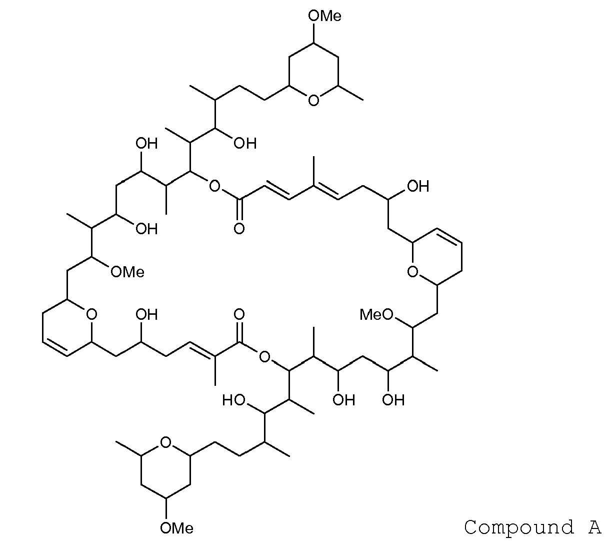

Compound A is a natural product isolated from a marine sponge, specifically a sponge of the species *Theonella swinhoei* (Class: Demospongiae, Subclass: Tetractinomorpha, Order: Lithistida, Suborder: Triaenosina, Family: Theonellidae). Said organism was harvested in Iles Glorieuses (northwest of Madagascar).

This sponge species is common in the western Pacific, in the Indian Ocean and in the Red Sea, being located off the coast up to 48 m deep. Specimens were found in:
- Kenya, Mombassa, off Shelly Beach, in the outer slope of the reef, at a depth of 12-16 m.
- North Kenya Banks (02° 25.5'S - 40° 52.5'E, at a depth of 48 m)
- Tulear, Ifaty area (Madagascar)
- Aldabra Island (northwest of Madagascar)
- Ternate, Celebes, Ambon, Manila, Formosa (Filipinas), Taiwan.

In addition, the analogs of said compound can be synthesized by any person skilled in the art by means of, for example, acid or base hydrolysis, oxidation, esterification, aldol condensation, ozonolysis, Wittig reaction, Horner-Emmons reaction, Sharpless epoxidation or Pictet-Spengler reaction. Compound A and its analogs can be synthesized, for example, by means of the described synthesis of the suitably protected Swinholide A and Misakinolide A monomer units, subsequently performing macrolactonization reactions which allow obtaining Compound A and analogs (See K-S. Yeung and I. Paterson. Angew. Chem. Int. Ed. 2002, 41, 4632-4653).

An important aspect of the compounds of the present invention is their bioactivity and in particular their cytotoxic activity. Therefore, the present invention provides novel pharmaceutical compositions of the compounds of general formula I having cytotoxic activity, as well as their use as antitumor agents. In addition, the present invention further provides pharmaceutical compositions comprising a compound of this invention, or a pharmaceutically acceptable salt, a derivative, a prodrug or a stereoisomer, in mixture with a pharmaceutically acceptable excipient or diluent.

Included as examples of pharmaceutical compositions are any solid (tablets, pills, capsules, granules, etc) or liquid (solutions, suspensions or emulsions) composition for oral, topical or parenteral administration. The pharmaceutical compositions containing the compounds of the present invention can also be formulated in the form of liposomes or nanospheres, of sustained release formulations or of any other conventional release system.

The administration of the compounds or compositions of the present invention can be performed by means of any of the usual methods such as intravenous infusion, oral preparations and/or intraperitoneal and intravenous administration. It is preferable that the used infusion times do not exceed 24 hours, being preferable from 1 to 12 hours, and even more preferable from 1 to 6 hours. Short infusion times are especially desired since they allow that the treatment is carried out without the need of having to spend the night in the hospital. Nevertheless, infusion times of from 12 to 24 hours and including more, can be used if necessary. The infusion can be carried out at suitable intervals, such as from 1 to 4 weeks.

The correct dosage of the compounds ranges according to the type of formulation used, the form of application and the situs, host and tumor to be treated. Other factors such as age, body weight, sex, diet, administration time, excretion rate, health condition of the host, combination of active ingredients, sensitivities in terms of reactions and disease severity must also be taken into account. The administration can be carried out continuously or periodically within the maximum tolerated dose.

The compounds and compositions of the present invention can be used together with other active ingredients in combined therapy. The other active ingredients can form part of the same composition or can be provided by means of a different composition, being administered at the same time or at different times.

### EXAMPLES

### Example 1: Description of the Organism and of the Harvest Site

Several samples of the sponge *Theonella swinhoei* were harvested by means of diving in Iles Glorieuses (northwest of Madagascar, west of the Indian Ocean), at a depth of 18 m, in November of 2003. The coordinates of the sampling site are the following: Latitude: 11°34'995'' S and, Longitude: 47°16'829'' E. The sea bed was rocky and sandy, and the substrate of the samples was rocks.

Description of the organism: massive sponge. The top surface supports several rounded openings (oscula). The surface is smooth and the consistency is hard, nevertheless the preserved specimens are fragile. The color of the live sponge is brown and its interior is a light beige color. The dermal membrane is uniformly porous. This species is easily characterized by the skeletal elements. The skeleton is formed by irregular bundles of strongyles, tangentially orientated close to the surface, and by scarce branched spicules (desmas). The choanosomal skeleton is made up of tetrazone spicules, which can be smooth or poorly tuberculated.

### Example 2: Isolating Compound A

A frozen sample (604 g) of the sponge of Example 1 was cut into pieces and extracted with H₂O (3 x 500 mL) and then with an MeOH:CH₂Cl₂ (50:50, 3 x 500 mL) mixture. The combined organic extracts were concentrated to give a residue (7.48 g) which was fractioned in VLC on Lichroprep RP-18 with a gradient from H₂O:MeOH to CH₂Cl₂. Compound A (5.8 mg) was isolated from the fraction eluted with MeOH which was purified by semipreparative HPLC (SymmetryPrep C-18, 7 µm, 7.8 mm x 150 mm, H₂O:CH₃CN gradient, UV detection) to give a fraction containing Compound A and which was again purified by semipreparative HPLC (SymmetryPrep C-18, 7 µm, 19 mm x 150 mm, gradient H₂O:CH₃CN, UV detection).

Compound A: MS (ESI) = 1362 (M+). MS (APCI)= 1345 (M+1-H₂O)+. ¹H and ¹³C NMR see Table 1.

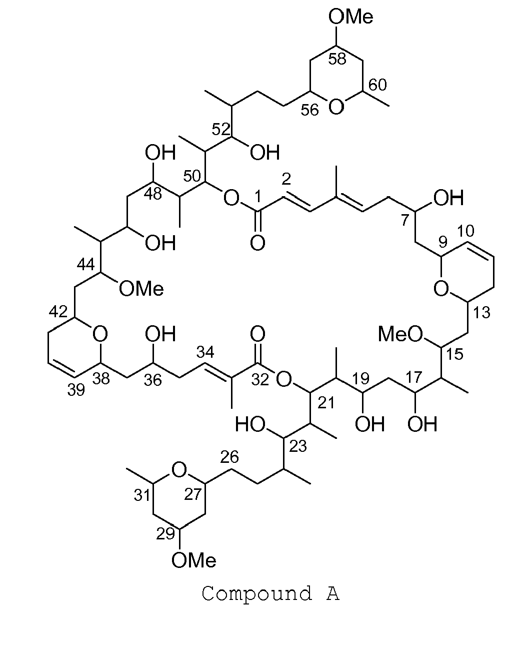

**Table 1. Data of ¹H and ¹³C NMR (CD₃OD) of compound A.**

| | ¹H, mult, *J*= Hz | ¹³C | COSY | HMBC |
|---|---|---|---|---|
| 1/32 | - | 170.7/171.1 | - | - |
| 2 | 5.91, d, 16.0 | 116.1 | H-3 | C-1, C-4 |
| 3 | 7.50, d, 16.0 | 152.4 | H-2 | C-1, C-2, C-4, 4-Me, C-5 |
| 4/33 | - | 135.6/129.9 | - | - |
| 4-Me/33-Me | 1.86, s/1.89, s | 12.7/13.22 | H-5/H-34 | C-3, 4-Me, C-5/C-32, C-33, C-34 |
| 5/34 | 6.16, t, 7.0/6.99, t, 6.0 | 140.9/142.1 | 4-Me, H-6/33-Me, H-35 | C-3, C-4, C-6, C-7/C-32, 33-Me, C-35, C-36 |
| 6/35 | 2.44, m/2.38, m | 38.8/38.6 | H-5, H-7/H-34, H-36 | C-4, C-5, C-7/C-33, C-34, C-36 |
| 7/36 | 4.02, m/4.08, m | 68.4/67.8 | H-6, H-8/H-35, H-37 | C-8/C-37 |
| 8/37 | 1.38, m/1.80, m^{a} | 41.58/41.63^{b} | H-7, H-9/H-36, H-38 | - |
| 9/38 | 4.49, d, 10.5 | 70.60/70.64^{c} | H-8/H-37, H-40 | C-10/C-39 |
| 10/39 | 5.68, m | 131.1 | H-11, H-13/H-40, H-41 | C-9, C-11, C-12/C-38, C-40, C-41 |
| 11/40 | 5.83, m | 125.05/125.20^{d} | H-10, H-12/H-38, H-39 | C-10/C-39 |
| 12/41 | 2.00, m | 32.37/32.44^{e} | H-11, H-13/H-39, H-42 | C-10, C-11/C-39, C-40 |
| 13/42 | 3.57, m | 65.5/65.7^{f} | H-10, H-12, H-14/H-41, H-43 | - |
| 14/43 | 1.58, m/1.82, m^{g} | 37.1/37.4^{h} | H-13, H-15/H-42, H-44 | C-13/C-42 |
| 15/44 | 3.78, m | 79.04/79.05ⁱ | H-14/H-43 | -/C-45 |
| 15-OMe/44-OMe | 3.33, s | 57.1/57.2^{j} | - | C-15/- |
| 16/45 | 1.58, m | 43.6/43.8^{k} | 16-Me/45-Me | C-15, C-18, C-19/C-47, C-48 |
| 16-Me/45-Me | 0.84, d, 5.5/0.86, d, 5.5^{l} | 9.05/9.40^{m} | H-16/H-45 | C-15, C-16, C-17/C-46, C-44, C-45 |
| 17/46 | 3.65, m/3.66, mⁿ | 73.8/74.2° | H-18/H-47 | - |
| 18/47 | 1.62, m/1.68, m^{p} | 39.0/39.2^{q} | H-17, H-19/H-46, H-48 | C-19/C-48 |
| 19/48 | 3.94, m | 70.9/70.8^{r} | H-18/H-47 | - |
| 20/49 | 1.90, m | 40.2/40.5 | H-21, 20-Me/H-50, 49-Me | C-21/C-50 |
| 20-Me/49-Me | 0.92, d, 7.5/0.95, d, 7.5^{s} | 9.4/9.5^{t} | H-20/H-49 | C-19, C-20, C-21/C-48, C-49, C-50 |
| 21/50 | 5.41, m | 76.5/76.0 | H-20/H-49 | C-19, C-20, C-22, 22-Me, C-23, C-32/C-1, C-48, C-49, 51-Me, C-51, C-52 |
| 22/51 | 2.03, m | 38.5/38.3 | 22-Me/51-Me | 22-Me, C-23/51-Me, C-52 |
| 22-Me/51-Me | 0.97, m | 10.1/9.9 | H-22/H-51 | C-22, C-23/C-51, C-52 |
| 23/52 | 3.13, dd, 2.0, 8.0 | 77.7/77.6^{u} | - | - |
| 24/53 | 1.73, m | 34.7 | 24-Me/53-Me | - |
| 24-Me/53-Me | 0.98, d, 7.0/0.99, d, 7.0^{v} | 18.0/18.1^{w} | H-24/H-53 | C-24, C-25/C-53, C-54 |
| 25/54 | 1.23, m/1.43, m^{x} | 25.30/25.35 | H-26/H-55 | C-24, C-26/C-53, C-55 |
| 26/55 | 1.94, m | 30.0 | H-25, H-27/H-54, H-56 | C-24, C-29/C-53, C-58 |
| 27/56 | 4.01, m | 73.03/73.06^{y} | H-26, H-28/H-57, H-55 | - |
| 28/57 | 1.52, m/1.88, m^{z} | 36.1 | H-27, H-29/H-56, H-58 | C-26, C-27, C-29, C-30/C-55, C-56, C-58, C-59 |
| 29/58 | 3.62, m | 74.5 | H-28, H-30/H-57, H-59 | - |
| 29-OMe/58-OMe | 3.36, s | 55.6 | - | C-29/C-58 |
| 30/59 | 1.11, q, 12.5/2.03, m^{az} | 40.0 | H-29, H-31/H-58, H-60 | C-28, C-29, C-31/C-57, C-58, C-60 |
| 31/60 | 3.76, m | 66.0 | H-30/H-59 | - |
| 31-Me/60-Me | 1.21, d, 6.5 | 22.1 | - | C-29, C-30, C-31/C-58, C-59, C-60 |

| | | | | |
|---|---|---|---|---|
| ^{a-az} the superscript in a box means that the values of the chemical displacements can be interchangeable. | | | | |

### Example 3: Bioassays of Antitumor Activity

The assays of antitumor activity allow detecting extracts or compounds with cytotoxic activity (cell death) or cytostatic activity (growth inhibition) in *in vitro* cultures of tumor cells of human origin.

**Cell Lines**

| Name | ATCC No. Species | Tissue | Characteristics |
|---|---|---|---|
| A549 | CCL-185 Human | Lung | "NSCL" lung cancer |
| HT29 | HTB-38 Human | Colon | Colonic adenocarcinoma Breast adenocarcinoma, |
| MDA-MB-231 | HTB-26 Human | Breast | Her2/neu+ (pleural effusion) |

### Study of Cell Growth Inhibition by means of Colorimetric Assay

The quantification of cell growth *in vitro* was carried out by means of a colorimetric assay with sulforhodamine B (SRB) (following an adaptation of the previously described method by Philip Skehan et al. 1990, J. Natl. Cancer Inst., 82:1107-1112).

This type of assay uses 96-well culture microplates, (Mosmann, 1983, Faircloth, 1988). Most of the cell lines used are obtained from the American Type Culture Collection (ATCC) and derived from different types of human tumors.

The cell cultures are maintained in DMEM culture medium (supplemented with 10% fetal bovine serum (FBS), 1% penicillin/streptomycin 1% glutamine) at 37 °C, 5% CO₂ and 98% humidity.

For the assays, the cells are trypsinized and seeded in 96-well microplates at different concentrations depending on the cell line, and are incubated for 24 hours in an active ingredient-free medium for their stabilization. The cultures are then treated (final concentration varies depending on the type of assay) with carrier (DMSO:DMEM, 1:1) or active ingredient. After 48 hours of exposure to the active ingredient, the antitumor effect is measured by means of the previously mentioned SRB method, which basically consists of: fixing the cells with a 1% glutaraldehyde solution (30 min, RT), washing of the fixer in PBS (3 washings, RT), staining the cultures with a 0.4% SRB solution (30 min, RT), washing of the colorant in 1% acetic acid solution (3 washings, RT), air-drying the plates and final extraction of the colorant with Tris buffer. The quantification of the assay is carried out by means of optical density reading of the plates in a spectrophotometric microplate reader at a single wavelength of 490 nm.

To estimate the cell growth from the obtained optical density values, an algorithm is applied (equivalent to the algorithm applied in the antitumor screening program of the NCI) which allows calculating growth percentage with respect to time zero (start of the experiment) both in the absence and in the presence of the active ingredient in question. The calculated parameters of cell response to the active ingredient are the following: GI₅₀ = concentration causing 50% growth inhibition, TGI = concentration causing total growth inhibition (cytostatic effect) and LC₅₀ = concentration causing 50% cell death (cytotoxic effect).

Table 2 shows the biological activity data of Compound A.

**Table 2. Data of antitumor activity (Molar)**

| | | Colon | Breast | NSCL (Lung) |
|---|---|---|---|---|
| | | HT29 | MDA-MB-231 | A549 |
| | GI₅₀ | 3.38E-7 | 8.08E-7 | 2.28E-7 |
| Compound A | TGI | 8.81E-7 | 2.35E-6 | 3.38E-7 |
| | LC₅₀ | 2.20E-6 | 4.77E-6 | 5.29E-7 |

## Claims

1. A compound of general formula I, or a pharmaceutically acceptable salt, a derivative, a prodrug or a stereoisomer thereof, 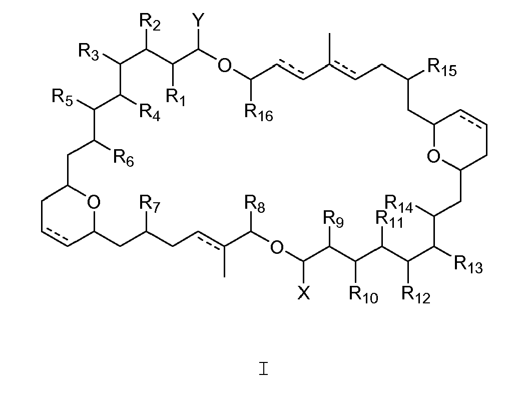 wherein,
R₁-₁₆ are groups independently selected from hydrogen, protected or non-protected hydroxyl, substituted or non-substituted C₁-C₂₄ alkyl, substituted or non-substituted C₂-C₂₄ alkenyl, substituted or non-substituted C₂-C₂₄ alkynyl, =O, ORₐ, OCORₐ, NRₐR_{b}, NRₐCOR_{b}, CONRₐR_{b}, CORₐ, COORₐ and halogen;
X and Y are groups independently selected from substituted or non-substituted C₁-C₂₄ alkyl, substituted or non-substituted C₂-C₂₄ alkenyl, substituted or non-substituted C₂-C₂₄ alkynyl, =O, ORₐ, OCORₐ, NRₐR_{b}, NRₐCOR_{b}, CONRₐR_{b}, CORₐ, COORₐ and halogen;
Rₐ and R_{b} are groups independently selected from hydrogen, halogen, substituted or non-substituted C₁-C₁₂ alkyl, substituted or non-substituted C₂-C₁₂ alkenyl, substituted or non-substituted C₂-C₁₂ alkynyl, substituted or non-substituted aryl and substituted or non-substituted heterocycle; and
the dotted line represents the optional presence of a double bond.

2. A compound according to claim 1, wherein R₁₋₁₆ are groups independently selected from hydrogen, protected or non-protected hydroxyl, ORₐ, OCORₐ, =O, NRₐR_{b}, NRₐCOR_{b}, halogen and substituted or non-substituted C₁-C₂₄ alkyl.

3. A compound according to claim 2, wherein R₁₋₇ and R₉₋₁₅ are groups independently selected from hydrogen, hydroxyl, C₁-C₆ alkyl and ORₐ.

4. A compound according to any one of claims 1 to 3, wherein R₈ and R₁₆ are =O.

5. A compound according to any one of claims 1 to 4, wherein X and Y are groups independently selected from substituted or non-substituted C₅-C₁₂ alkyl and substituted or non-substituted C₅-C₁₂ alkenyl.

6. A compound according to claim 5, wherein X and Y are groups independently selected from C₅-C₁₂ alkyl and C₅-C₁₂ alkenyl, which are substituted by one or several of the following substituents: OH, OR', NHCOR' and substituted or non-substituted heterocycle, wherein R' is selected from H, OH, NO₂, NH₂, SH, CN, halogen, =O, C(=O)H, C(=O)alkyl, COOH, substituted or non-substituted C₁-C₁₂ alkyl, substituted or non-substituted C₂-C₁₂ alkenyl, substituted or non-substituted C₂-C₁₂ alkynyl and substituted or non-substituted aryl and substituted or non-substituted heterocycle.

7. A compound according to claim 6, wherein X and Y are C₅-C₁₂ alkyl groups, which are substituted by one or several hydroxyl groups and/or substituted heterocycles.

8. A compound according to any of claims 1 to 7, having the following formula:

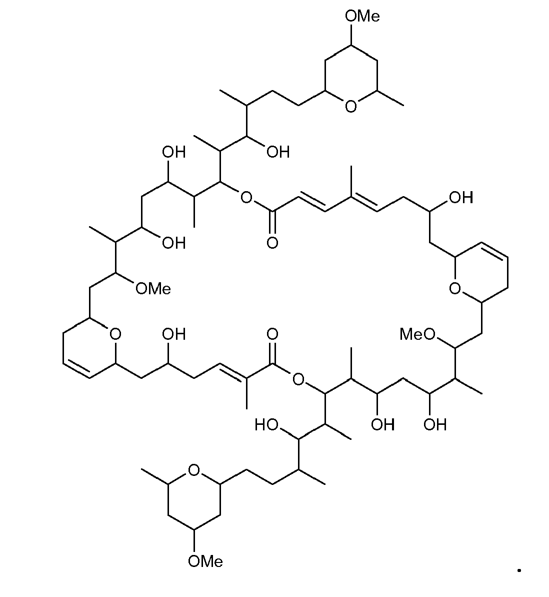

9. A process for obtaining a compound as it is defined in any one of the previous claims, comprising its extraction and isolation from an organism of the species *Theonella swinhoei.*

10. A pharmaceutical composition comprising a compound according to any one of claims 1 to 8, or a pharmaceutically acceptable salt, a derivative, a prodrug or a stereoisomer thereof, in mixture with a pharmaceutically acceptable excipient or diluent.

11. The compound according to any of claims 1 to 8, or a pharmaceutically acceptable salt, a derivative, a prodrug or a stereoisomer thereof, for its use as a medicament.

12. A use of a compound according to any of claims 1 to 8, or a pharmaceutically acceptable salt, a derivative, a prodrug or a stereoisomer thereof for the preparation of a medicament aimed at treating cancer.
